# EUROPÄISCHE PATENTSCHRIFT

(11) **EP 2 179 276 B1**
(45) Veröffentlichungstag und Bekanntmachung des Hinweises auf die Patenterteilung: **09.11.2011**
(21) Anmeldenummer: 08803035.8
(22) Anmeldetag: 14.08.2008
(51) Int. Cl.: G01N 27/414, G01N 33/00

(54) **Verfahren zur Bestimmung von Gaskomponenten in Gasgemischen**
Method to determine gaseous components in gas mixtures
Procédé de détermination des components gazeuses dans mixtures de gaz

(30) Priorität: 22.08.2007 DE 102007039567
(43) Veröffentlichungstag der Anmeldung: 28.04.2010
(73) Patentinhaber: Robert Bosch GmbH, 70442 Stuttgart (DE)
(72) Erfinder: LIEMERSDORF, Dirk, 70839 Gerlingen (DE); FIX, Richard, 70839 Gerlingen (DE)
(86) Internationale Anmeldenummer: PCT/EP2008/060687
(87) Internationale Veröffentlichungsnummer: WO 2009/024526

(56) Entgegenhaltungen:
- DE-A1-102005 033 226
- DE-A1-102005 033 226
- JP-A- 57 007 549
- US-A1- 2004 112 764
- US-A1- 2005 097 941
- FILIPPINI D ET AL: "Field-effect NO2 sensors with group 1B metal gates" SENSORS AND ACTUATORS B, ELSEVIER SEQUOIA S.A., LAUSANNE, CH, Bd. 81, Nr. 1, 15. Dezember 2001 (2001-12-15), Seiten 83-87, XP004324024 ISSN: 0925-4005
- D. YU. ZEMLYANOV, A. NAGY, R. SCHLÖGL: "The reaction of silver with NO/O2" APPLIED SURFACE SCIENCES, Bd. 133, 1998, Seiten 171-183, XP002503397

## Beschreibung

### Stand der Technik

Die Erfindung bezieht sich auf eine Verfahren zur Bestimmung von Gaskomponenten in Gasgemischen, insbesondere in Abgasen von Verbrennungsmotoren.

Zur Bestimmung von Gaskomponenten in Gasgemischen werden u.a. Feldeffekttransistoren eingesetzt. Dabei reagiert bspw. eine Gate-Elektrode des Feldeffekttransistors sensitiv auf zu bestimmende Gaskomponenten, wodurch es zu einer Veränderung einer an der Gate-Elektrode anliegenden Steuerspannung kommt. Die dabei auftretende Veränderung des zwischen Source-und Drain-Elektroden des Feldeffekttransistors resultierenden Stromflusses wird detektiert und einer Konzentration der Gaskomponenten zugeordnet.

Ein derartiger Gassensor ist bspw. der DE 10 2005 010 454 A1 zu entnehmen. Dabei weist die Gate-Elektrode eine saure oder basische Beschichtung auf, die die Sensitivität des Gassensors auf brandrelevante Gase erhöht. Nachteilig ist, dass nur pH-aktive Gase erfasst werden können. Eine Bestimmung von Abgasbestandteilen ohne nennenswerte Querempfindlichkeit auf Kohlenwasserstoffe ist nicht möglich.

Aus der US 2004/0112764 A1 ist eine Sensorvorrichtung zur Erfassung von Kohlenwasserstoffgasen in ölgefüllten Hochspannungsanlagen bekannt, bei der eine gasempfindliche, katalytische Metallschicht als Gate-Elektrode auf einem Halbleitersubstrat angeordnet ist.

Aus der Sensors and Actuators B 81 (2001), Filippini et al: "Field-effect NO2 sensors with group 1B metal gates" ist eine Messeinrichtung bekannt, bei der Stickstoffdioxid mittels einem Gate aus Kupfer, Silber oder Gold als Gate eines MOS-Schalters gemessen wird.

### Offenbarung der Erfindung

Der vorliegenden Erfindung liegt die Aufgabe zugrunde, ein Verfahren zum Betrieb eines Gassensors zur Verfügung zu stellen, das die unabhängige Bestimmung von Stickoxiden ohne nennenswerte Querempfindlichkeiten auf im zu messenden Gasgemisch vorliegende Kohlenwasserstoffe ermöglicht.

### Vorteile der Erfindung

Ein Verfahren mit den kennzeichnenden Merkmalen des unabhängigen Anpruchs löst in vorteilhafter Weise die der Erfindung zugrunde liegende Aufgabe. Dabei weist der Gassensor eine silberhaltige Schicht auf und ermöglicht dabei als Bestandteil einer Gate-Elektrode eines Feldeffekttransistors die Bestimmung von Stickoxiden in Gasgemischen weitgehend ohne Querempfindlichkeiten zu in einem Gasgemisch ebenfalls vorliegenden Kohlenwasserstoffen.

So ist von Vorteil, wenn die gassensitive Schicht zusätzlich ein Element der Münzmetalle Rhodium, Iridium, Palladium und Platin enthält, da auf diese Weise der Schmelzpunkt der gassensitiven Schicht erhöht und somit deren Strukturstabilität verbessert wird. Weiterhin kann auf diese Weise eine deutlich höhere Oxidationsstabilität der gassensitiven Schicht erreicht werden. Darüber hinaus wird durch die Kombination mehrerer Elemente mit Silber als Material der gassensitiven Schicht die Ansprechzeit, Sensitivität und Selektivität des Sensors günstig beeinflusst.

Weiterhin ist die gassensitive Schicht als Beschichtung einer metallischen Gate-Elektrode eines Feldeffekttransistors ausgeführt, wobei die gassensitive Schicht und die Gate-Elektrode aus verschiedenen Materialien ausgeführt sind und die gassensitive Schicht in direktem physischem Kontakt mit dem metallischen Elektrodenmaterial der Gate-Elektrode steht. Auf diese Weise lässt sich das Potenzial der Gate-Elektrode in Abhängigkeit von der Zusammensetzung des zu messenden Gasgemischs besonders effektiv beeinflussen, ohne dass das Elektrodenmaterial der Gate-Elektrode selbst aus dem gassensitiven Material ausgeführt werden muss. Dies ermöglicht eine größere Bandbreite der Materialauswahl für die Gate-Elektrode bzw. die gassensitive Schicht.

In einer besonders vorteilhaften Ausführungsform der vorliegenden Erfindung ist ein erster und ein zweiter Feldeffekttransistor vorgesehen, wobei der erste Feldeffekttransistor die gassensitive Schicht umfasst und der zweite Feldeffekttransistor als Referenzsensor keine derartige gassensitive Schicht aufweist oder eine gassensitive Schicht anderer Zusammensetzung. Auf diese Weise werden mittels der beiden Feldeffekttransistoren zwei unterschiedliche Messsignale erhalten, wobei durch Differenzbildung der beiden Messsignale beispielsweise die Konzentration an Kohlenwasserstoffen im zu bestimmenden Gasgemisch ermittelt werden kann.

Das beanspruchte Verfahren hat unter anderem den Vorteil, dass die Temperaturabhängigkeit der Messsignale des erfindungsgemäßen Gassensors zur Bestimmung von Gaskomponenten ausgenutzt werden kann. So wird zunächst auf eine erste gegenüber Raumtemperatur erhöhte Temperatur erwärmt und bei dieser Temperatur eine erste Messung durchgeführt, bei der ein erstes Messsignal erhalten wird. Danach wird auf eine zweite, gegenüber der ersten Temperatur erhöhte Temperatur erwärmt und eine zweite Messung durchgeführt, die zu einem zweiten Messsignal führt, das Abweichungen gegenüber dem ersten Messsignal zeigt. Unter Verwendung eines entsprechenden Kennfeldes kann durch Differenzbildung beider Signale auf die Konzentration bestimmter Gaskomponenten geschlossen werden. So lässt sich erfindungsgemäß ein bei der ersten erhöhten Temperatur erhaltenes Messsignal einer Stickoxidkonzentration zuordnen und ein bei der zweiten Bestimmung erhaltenes Messsignal der Summe aus Stickoxid-, Ammoniak- und/oder Wasserstoffkonzentration im Gasgemisch. Durch einfache Diiierenzbildung kann die Summe der Konzentrationen an Ammoniak und/oder Wasserstoff im Gasgemisch bestimmt werden.

### Kurze Beschreibung der Zeichnungen

Ein Ausführungsbeispiel der Erfindung ist in der Zeichnung dargestellt und in der nachfolgenden Beschreibung näher erläutert. So zeigt:
- Figur 1:: eine schematische Schnittdarstellung eines Gassensors gemäß einer ersten Ausführungsform der vorliegenden Erfindung,
- Figur 2:: das Messsignal eines Gassensors in einer abweichenden Ausfllhrungsform bei Zufuhr verschiedener Messgase bei einer Temperatur von 350 °C,
- Figur 3:: das Messsignal eines gemäß Figur 1 ausgeführten Gassensors bei Zufuhr verschiedener Messgase bei einer Temperatur von 350 °C,
- Figur 4:: das Messsignal eines gemäß Figur 1 ausgeführten Gassensors bei Zufuhr verschiedener Messgase bei einer Temperatur von 500 °C und
- Figur 5: das Messsignal eines Gassensors gemäß einem zweiten Austührungsbeispiel bei Zufuhr verschiedener Messgase bei einer Temperatur von 400 °C.

### Ausführungsformen der Erfindung

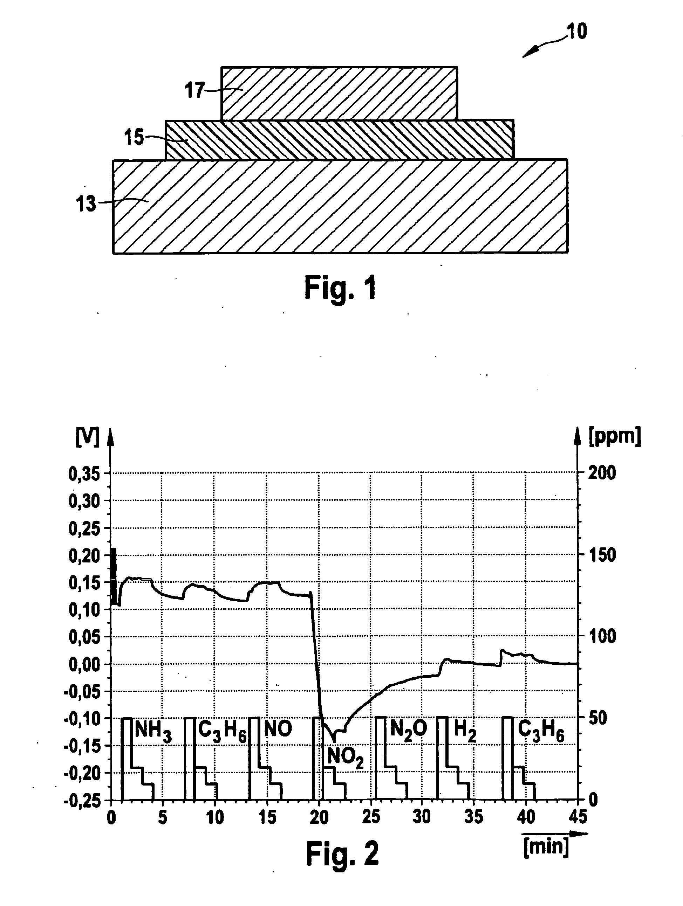

Ein erfindungsgemäß ausgebildeter Gassensor 10 umfasst, wie in Figur 1 dargestellt, ein Substrat 13, welches aus einem Halbleitermaterial gefertigt ist. Ein bevorzugtes Halbleitermaterial ist Galliumnitrid; wobei sich neben Galliumnitrid als Halbleitermaterial jedoch auch Aluminiumnitrid, Galliumaluminiumnitrid und Siliziumcarbid eignet. Galliumnitrid ist ein Ill-V-Verbindungshalbleiter, der z.B. für Hochleistungs- und Hochfrequenz-Feldeffekttransistoren verwendet wird. Elektronische Bauteile mit einem Substrat aus Galliumnitrid eignen sich prinzipiell für Betriebstemperaturen bis etwa 700°C. Somit können derartige Bauelemente auch bei Hochtemperaturanwendungen, z.B. zur Gassensorik im Abgasstrom von Kraftfahrzeugen eingesetzt werden.

Auf das Substrat 13 aus Halbleitermaterial ist vorzugsweise zunächst eine elektrische Isolationsschicht 15 aufgebracht. Die elektrische Isolationsschicht 15 ist vorzugsweise aus dem gleichen Material hergestellt wie die Passivierung eines den Gassensor 10 umfassenden Chips. Als Material für die elektrische Isolationsschicht 15 eignet sich z.B. Si₃N₄. Die Dicke der elektrischen Isolationsschicht 15 liegt vorzugsweise im Bereich von 1 bis 100 nm.

Im Allgemeinen wird das Material der elektrischen Isolationsschicht 15 auf dem Substrat 13 aus dem Halbleitermaterial abgeschieden. So ist z.B. eine In-situ-Abschcidung von Si₃N₄ direkt nach dem Wachstum der Halbleiter-Oberfläche aus Galliumnitrid möglich. Alternativ ist es jedoch auch möglich, dass die elektrische Isolationsschicht 15 eine Ex-situ-abgeschiedene CVD (chemical vapor deposition) Si₃N₄- oder SiC-Schicht ist.

Der Gassensor 10 ist alls Feldeffekttransistor ausgeführt, also dient die elektrische Isolationsschicht 15 der Isolierung einer Gate-Elektrode 17, um Gate-Leckströme und damit Elektro-Migration zu minimieren, den elektrischen Betrieb zu stabilisieren und eine einfache Signalauswertung sicherzustellen. Dabei ist die elektrische Isolationsschicht 15 als Zwischenschicht zwischen dem Substrat 13 und der Gate-Elektrude 17 vorgesehen.

Zwischen der elektrischen Isolationsschicht 15 und der Gate-Elektrode 17 kann zusätzlich eine nicht dargestellte Diffusionssperrschicht mit einer Dicke im Bereich von 1 bis 300 nm aus einem elektrisch leitfähigen Material vorgesehen sein.

Die Gate-Elektrode 17 ist vorzugsweise aus einem silberhaltigen, elektrisch leitfähigen Material ausgeführt. Dabei hat die Materialauswahl für das Elektrodenmaterial der Gate-Elektrode 17 einen wesentlichen Einfluss auf die Selektivität des vorliegenden Gassensors.

Die Funktionsweise eines als Feldeffekttransistor ausgebildeten Gassensors beruht darauf, dass das Substrat 13 mit einer Source- und einer Drain-Elektrode kontaktiert wird und der zwischen Source-und Drain-Elektrode fließende elektrische Strom durch ein an der Gate-Elektrode 17 anliegendes Potenzial beeinflusst werden kann. Dabei wird an die Gate-Elektrode 17 vorzugsweise ein geeignetes konstantes Potenzial angelegt, dessen Höhe jedoch aufgrund der Gassensitivität der Gate-Elektrode 17 in Abhängigkeit von der Konzentration der zu bestimmenden Gaskomponenten in einem die Gate-Elektrode 17 umgebenden Gasgemisch beeinflusst wird. Als Messsignal wird der sich aufgrund des variablen Gate-Potenzials verändernde elektrische Strom zwischen Source- und Gate-Elektrode herangezogen.

Übliche Gate-Elektroden, beispielsweise aus Platin, Gold, Iridium oder Palladium, zeigen, wie in Abbildung 2 erkennbar, ein Messsignal, das bei Zutritt von Stickstoffdioxid besonders ausgeprägt ausfällt. Ein derart ausgeführter Gassensor zeigt jedoch eine in Figur 2 erkennbare Querempfindlichkeit auf andere übliche Inhaltsstoffe von Verbrennurgsabgasen wie Ammoniak, Kohlenwasserstoff, Stickstoffmonoxid, Distickstoffmonoxid und Wasserstoff. Dabei ist in Figur 2 der Gehalt an zu detektierenden Gaskomponenten in einem Messgas (Stickstoff) in ppm über einer Zeitachse aufgetragen, wobei die linke Abszisse das an der Gate-Elektrode dabei resultierende Gate-Potenzial in Volt anzeigt. Die Messung wurde bei einer Umgebungstemperatur von 350 °C durchgeführt. Es ist erkennbar, dass die Empfindlichkeit eines Sensors dieser Ausfiihrungsform auf andere übliche Abgasbestandteile außer Stickstoffdioxid im wesentlichen mit identischer Empfindlichkeit anspricht.

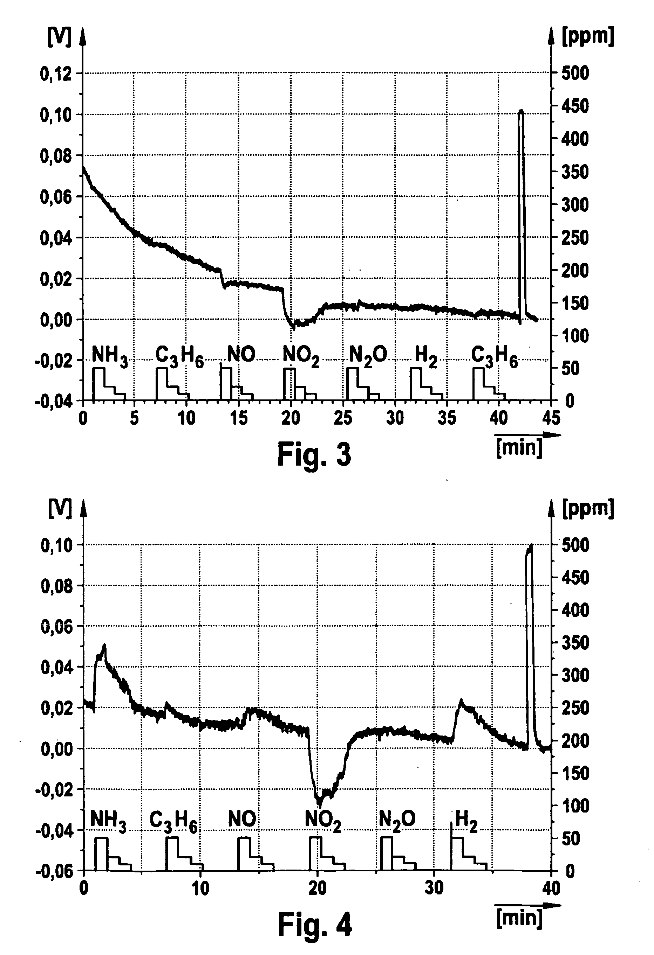

Wird jedoch eine Gate-Elektrode 17 verwendet, die aus einem silberhaltigen, elektrisch leitfähigen Material gebildet ist, so wird ein abweichendes Messsignal, wie es beispielsweise in Figur 3 dargestellt ist, erhalten. Es ist deutlich zu erkennen, dass das Messsignal des Gassensors eine deutliche Empfindlichkeit auf Stickstoffdioxid und eine geringe, jedoch ebenfalls spürbare Empfindlichkeit auf Stickstoffinonoxid zeigt, jedoch keine Querempfindlichkeiten auf weitere übliche Gaskomponenten eines Verbrennungsabgases wie Ammoniak, Kohlenwasserstoffe, Distickstoffinonoxid und Wasserstoff. Dabei ist in Figur 3 der Gehalt an zu detektierenden Gaskomponenten in einem Messgas (Stickstoff) in ppm über einer Zeitachse aufgetragen, wobei die linke Abszisse das an der Gate-Elektrode dabei resultierende Gate-Potenzial in Volt anzeigt. Die in Figur 3 dargestellte Messung wurde ebenfalls bei einer Messtemperatur von 350 °C durchgeführt.

Eine ähnlich ausgeprägte Selektivität auf Stickoxide lässt sich bei Verwendung von silbcrhaltigen elektrisch leitfähigen Materialien erreichen, die zusätzlich ein Element der Münzmetalle Rhodium, Iridium, Palladium und Platin enthält, wobei Platin bevorzugt ist. Dabei kann das Material vorzugsweise als Cermet ausgeführt werden, um eine gute Anbindung an das Material der Isolationsschicht 15 zu gewährleisten. Das Material der Gate-Elektrode 17 kann darüber hinaus weitere Elemente enthalten und bspw. als ternäre oder quarternäre Legierung ausgebildet sein.

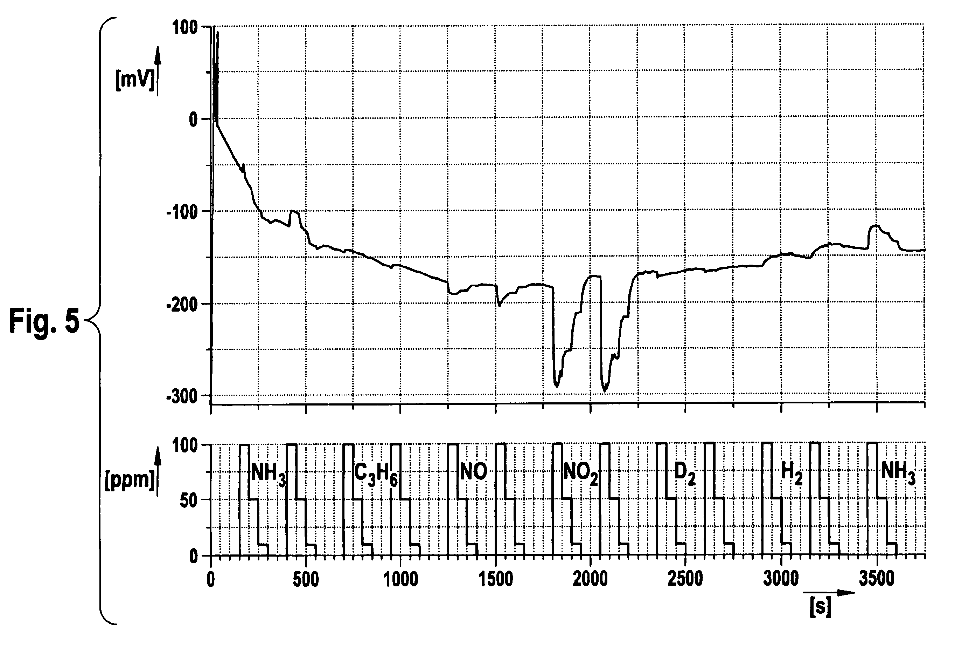

Eine weitere Möglichkeit besteht darin, die Gate-Elektrode 17 mit einer nicht dargestellten Beschichtung aus dem vorgenannten silber- bzw. silber- und platinhaltigen gassensitiven Material zu versehen und die Gate-Elektrode 17 selbst aus einem gängigen Elektrodenmaterial auszuführen. Auch dieser Aufbau eines Gassensors führt zur gewünschten Selektivität auf Stickoxide unter Diskriminierung anderer Abgaskomponenten wie Ammoniak, Kohlenwasserstoffe. Distickstoffmonoxid oder Wasserstoff, ohne dass das Material der Gate-Elektrode 17 hinsichtlich seines Materials einer Beschränkung unterliegt. Das Messsignal eines derart ausgeführten Gassensors ist in Figur 5 dargestellt. Dabei ist der Gehalt an zu detektierenden Gaskomponenten in einem Messgas (Stickstoff) in ppm über einer Zeitachse aufgetragen, wobei die linke Abszisse das an der Gate-Elektrude dabei resultierende Gate-Potenzial in Volt anzeigt. Die Messung wurde bei einer Umgebungstemperatur von 400 °C durchgeführt.

Die Aufbringung der Metallisierung aus Silber bzw. Platin auf die Gate-Elcktrode 17 erfolgt vorzugsweise mittels einer nasschemischen Aufbringung von kolloidalem Platin bzw. Silber in Lösung. Der resultierende Gasscnsor umfasst eine Beschichtung der Gate-Elektrode 17 mit Nanopartikeln aus Silber und Platin und zeigt im wesentlichen keine Sensitivität auf Kohlenwasserstoffe, die in der Messung exemplarisch durch C₃H₆ vertreten sind, oder auf Wasserstoff. Er zeigt darüber hinaus nur eine geringfügige Querempfindlichkeit auf Ammoniak.

Auffällig ist jedoch die starke Selektivität des Gassensors zwischen NO und NO₂. So ist der Gassensor deutlich sensitiver auf NO₂ als auf NO.

Ein mögliches Betriebsverfahren des beschriebenen Gassensors besteht darin, eine erste Messung beispielsweise in einem gegenüber Raumtemperatur erhöhten Temperaturbereich von beispielsweise 200 bis 500 Grad Celsius, vorzugsweise von 200 bis 400 Grad Celsius, insbesondere bei 350 Grad Celsius durchzuführen, wobei, bedingt durch die beschriebene gassensitive Schicht, der Gassensor ein selektives Messsignal auf Stickoxide zeigt. Dann wird in einem zweiten Schritt die Temperatur des Gassensors auf eine zweite gegenüber der ersten erhöhten Temperatur erhöht, beispielsweise auf eine Temperatur über 400 bis 600 Grad Celsius, vorzugsweise von 450 bis 550 Grad Celsius, insbesondere auf 500 Grad Celsius, wobei der Gassensor, wie in Figur 4 dargestellt, neben einer Selektivität auf Stickoxide zusätzlich auf wasserstoffhaltige Abgaskomponenten wie Ammoniak und Wasserstoff anspricht. Dabei ist in Figur 4 der Gehalt an zu detektierenden Gaskomponenten in einem Messgas (Stickstoff) in ppm über einer Zeitachse aufgetragen, wobei die linke Abszisse das an der Gate-Elektrode dabei resultierende Gate-Potenzial in Volt anzeigt. Die Messung wurde bei einer Umgebungstemperatur von 500 °C durchgeführt.

Zur Einstellung der ersten bzw. zweiten erhöhten Temperatur kann der Gassensor 10 ein nicht dargestelltes Heizelement umfassen, das bspw. als Widerstandsleiterbahn ausgeführt ist und in das Substrat 13 des Gassensors integriert werden kann.

Auf diese Weise kann bei einer Messung bei einer ersten erhöhten Temperatur ein selektives Signal auf Stickoxide gewonnen werden und bei einer Messung im Bereich der zweiten erhöhten Temperatur ein Messsignal, das zusätzlich eine Abhängigkeit von wasserstoffhaltigen Gaskomponenten des zu messenden Gasgemischs zeigt. Auf diese Weise kann, beispielsweise durch Differenzbildung, die selektive Bestimmung von wasserstoffhaltigen Komponenten im Abgas trotz Vorliegen von Stickoxiden zuverlässig erfolgen. Darüber hinaus kann durch Auswertung des Messsignals im Bereich der ersten erhöhten Temperatur ein von der Stickoxidkonzentration in dem zu messenden Gasgemisch allein abhängiges Messsignal gewonnen werden.

Wird alternativ oder zusätzlich der beschriebene Gassensor mit einem zweiten Gassensor gegebenenfalls auf einem gemeinsamen Substrat kombiniert, so ist dieser zweite Gassensor, dessen Messsignal in Figur 2 dargestellt ist, bei Verwendung einer Gate-Elektrode, die unter anderem Platin, Gold, Iridium oder Palladium, jedoch kein Silber umfasst, sensitiv sowohl auf wasserstoffhaltige Abgaskomponenten wie auch auf Kohlenwasserstoffe und Stickoxide. Wird nun eine Messung mit einem kombinierten Gassensor, der einen ersten Feldeffekttransistor mit silberhaltiger Gate-Elektrode und einen zweiten Feldeffekttransistor mit nicht silberhaltiger Elektrode aufweist, im Bereich der ersten erhöhten Temperatur durchgeführt, so wird mittels des ersten Feldeffekttransistors ein lediglich von Stickoxiden abhängiges Messsignal erhalten, während mittels des Referenzfeldeffekttransistors ein von Stickoxiden, wasserstoffhaltigen Gaskomponenten und Kohlenwasserstoffen abhängiges Messsignal generiert wird. Die Differenzbildung beider Signale ermöglicht die Bestimmung der Gesamtkonzentration aus wasserstoffhaltigen Gaskomponenten und Kohlenwasserstoffen im Gasgemisch.

Wird in einem zweiten Schritt eine Messung im Bereich der zweiten erhöhten Temperatur durchgeführt, so kann dort ein weiteres Messsignal des ersten Feldeffekttransistors gewonnen werden, das die Gesamtkonzentration aus Stickoxiden und wasserstoffhaltigen Gaskomponenten umfasst. Durch Differenzbildung dieses dritten Messsignals mit den bei der ersten Messung gewonnenen Messsignalen kann durch Differenzbildung die Konzentration von Kohlenwasserstoffen ohne Querempfindlichkeit auf Stickoxide und wasserstoffhaltige Gaskomponenten gewonnen werden.

## Patentansprüche

1. Verfahren zur Bestimmung von Gaskomponenten in Gasgemischen, insbesondere in Abgasen von Verbrennungsmotoren, mittels eines Gassensors.
mit einer auf einem Substrat (13) aus einem Halbleitermaterial aufgebrachten und dem zu messenden Gasgemisch ausgesetzten gassensitiven Schicht, wobei die gassensitive Schicht Silber enthält, wobei das Halbieitennaterial Bestandteil eines Feldeffekttransistors ist und wobei die gassensitive Schicht Bestandteil einer Gate-Elektrode (17) des Feldeaekttransistors ist, wobei die gassensitive Schicht als Beschichtung einer metallischen Gate-Elektrode (17) ausgeführt ist, wobei die gassensitive Schicht und die Gato-Elektrode (17) aus verschiedenen Materialien ausgerührt sind und die gassensitive Schicht in direktem physischen Kontakt mit dem metallischen Elektrodenmaterial der Gate-Elektrode (17) steht,
wobei der Gassensor zunächst auf eine erste gegenüber Raumtemperatur erhöhte Temperatur erwärmt wird und bei dieser Temperatur eine erste Bestimmung der Gaskomponenten erfolgt und wobei in einem zweiten Schritt der Gassensor auf eine zweite gegenüber Raumtemperatur erhöhte Temperatur erwärmt wird, die höher als die erste erhöhte Temperatur ist, und bei dieser zweiten erhöhten Temperatur eine zweiten Bestimmung der Gaskomponenten erfolgt, **dadurch gekennzeichnet, dass** ein während der ersten Bestimmung erhaltenes Messsignal einer Stictcoxidkonzentiation zugeordnet wird und dass ein während der zweiten Bestimmung erhaltenes Messsignal einer Summe der Stickoxid-, Ammoniak- und Wasserstoffkonzentration im Gasgemisch zugeordnet wird.

2. Verfahren nach Anspruch 1, **dadurch gekennzeichnet, dass** das Verfahren mit mindestens zwei Fetdeffekttreasistoren durchgeführt wird und dass mittels der mindestens zwei Feldeffekttransistoren jeweils ein Messsignal generiert und beide Messsignale miteinander verglichen werden.

3. Verfahren nach Anspruch 2, **dadurch gekennzeichnet, dass** eine Differenz beider Messsignale gebildet wird und die Differenz einer Kohlenwasserstoffkonzentration im Gasgemisch zugeordnet wird.

4. Verwendung eines Verfahrens nach einem der Ansprüche 1 bis 3 zur überwachung der Funktionstuchtigkeit eines NOx-Speichetkatalysators oder eines SCR-Abgasnachbehandlungssystems.

## Claims

1. Method for determining gas components in gas mixtures, in particular in exhaust gases of internal combustion engines, by means of a gas sensor, with a gas-sensitive layer applied to a substrate (13) of a semiconductor material and exposed to the gas mixture to be measured, the gassensitive layer containing silver, the semiconductor material being a component part of a field-effect transistor and the gas-sensitive layer being a component part of a gate electrode (17) of the field-effect transistor, wherein the gassensitive layer is formed as a coating of a metallic gate electrode (17), wherein the gassensitive layer and the gate electrode (17) are formed from different materials and the gassensitive layer is in direct physical contact with the metallic electrode material of the gate electrode (17), wherein the gas sensor is first heated to a first temperature that is elevated with respect to room temperature and a first determination of the gas components is performed at this temperature and wherein, in a second step, the gas sensor is heated to a second temperature that is elevated with respect to room temperature and is higher than the first elevated temperature and a second determination of the gas components is performed at this second elevated temperature, **characterized in that** a measuring signal that is obtained during the first determination is assigned to a nitrogen-oxide concentration and **in that** a measuring signal that is obtained during the second determination is assigned to a sum of the nitrogen-oxide, ammonia and hydrogen concentration in the gas mixture.

2. Method according to Claim 1, **characterized in that** the method is carried out with at least two field-effect transistors and **in that** a measuring signal is respectively generated by means of the at least two field-effect transistors and the two measuring signals are compared to one another.

3. Method according to Claim 2, **characterized in that** a difference between the two measuring signals is formed and the difference is assigned to a hydrocarbon concentration in the gas mixture.

4. Use of a method according to one of Claims 1 to 3 for monitoring the functional capability of a NOx storage catalyst or an SCR emission control system.

## Revendications

1. Procédé pour déterminer les composantes gazeuses dans des mélanges gazeux, notamment dans les gaz d'échappement des moteurs à combustion interne, au moyen d'un détecteur de gaz
comprenant une couche sensible au gaz appliquée sur un substrat (13) constitué d'un matériau semiconducteur et exposée au mélange gazeux à mesurer, la couche sensible au gaz contenant de l'argent, le matériau semiconducteur faisant partie d'un transistor à effet de champ et la couche sensible au gaz faisant partie d'une électrode de gâchette (17) du transistor à effet de champ, la couche sensible au gaz étant réalisée sous forme de revêtement d'une électrode de gâchette (17) métallique, la couche sensible au gaz et l'électrode de gâchette (17) étant réalisées dans des matériaux différents et la couche sensible au gaz étant en contact physique direct avec le matériau d'électrode métallique de l'électrode de gâchette (17),
le capteur de gaz étant tout d'abord réchauffé à une première température accrue par rapport à la température ambiante et une première détermination des composantes gazeuses ayant lieu à cette température et, dans une deuxième étape, le capteur de gaz étant réchauffé à une deuxième température accrue par rapport à la température ambiante, laquelle est supérieure à la première température accrue, et une deuxième détermination des composantes gazeuses ayant lieu à cette deuxième température accrue, **caractérisé en ce qu'**un signal de mesure obtenu pendant la première détermination est associé à une concentration d'oxyde d'azote et **en ce qu'**un signal de mesure obtenu pendant la deuxième détermination est associé à une somme de la concentration d'oxyde d'azote, d'ammoniac et d'hydrogène dans le mélange gazeux.

2. Procédé selon la revendication 1, **caractérisé en ce que** le procédé est mis en oeuvre avec au moins deux transistors à effet de champ et **en ce qu'**un signal de mesure est à chaque fois généré au moyen des au moins deux transistors à effet de champ et les deux signaux de mesure sont comparés entre eux.

3. Procédé selon la revendication 2, **caractérisé en ce qu'**une différence entre les deux signaux de mesure est calculée et une concentration d'hydrocarbures dans le mélange gazeux est associée à la différence.

4. Utilisation d'un procédé selon l'une des revendications 1 à 3 pour surveiller l'aptitude fonctionnelle d'un catalyseur à accumulateur de NOx ou d'un système de post-traitement des gaz d'échappement SCR.
